# EUROPEAN PATENT APPLICATION

(11) **EP 3 705 129 A1**
(43) Date of publication of application: **09.09.2020**
(21) Application number: 18872129.4
(22) Date of filing: 11.05.2018
(51) Int. Cl.: A61K 36/736, A61K 36/62, A23L 33/105

(54) **COMPOSITION FOR PREVENTING, AMELIORATING OR TREATING OBESITY AND METABOLIC DISEASES, COMPRISING COMPLEX EXTRACT FROM PEACH BLOSSOM AND LOTUS LEAF**

(30) Priority: 31.10.2017 KR 20170143819
(71) Applicant: University-Industry Cooperation Group of Kyung Hee University, Yongin-si, Gyeonggi-do 17104 (KR); Neumed, Seoul 02440 (KR)
(72) Inventor: KIM, Hocheol, Seoul 02448 (KR); SONG, Jungbin, Seoul 04714 (KR); LEE, Yoon Hee, Seoul 04206 (KR)
(74) Representative: Icosa
(86) International application number: PCT/KR2018/005429
(87) International publication number: WO 2019/088381

(57) **Abstract**

Provided are a composition for preventing, improving, or treating obesity or metabolic diseases, the composition including a combined extract of peach blossom and lotus leaf or a fraction thereof as an active ingredient, use thereof, and a treatment method thereof. The combined extract of peach blossom and lotus leaf of the present invention or the fraction thereof has excellent efficacy against obesity or metabolic diseases and has no side effects because it is derived from natural products, and therefore, it may be variously applied to a pharmaceutical composition for preventing or treating metabolic diseases or a food composition for preventing or improving obesity or metabolic diseases, or applied to preparation of drugs or to a treatment method.

## Description

### [Technical Field]

The present invention relates to a pharmaceutical composition for preventing or treating obesity or metabolic diseases and a food composition for preventing or improving obesity or metabolic diseases, each including a combined extract of peach blossom and lotus leaf or a fraction thereof as an active ingredient.

### [Background Art]

In modern society, change to a meat-based diet leads to excessive caloric intake and lack of physical exercise, and thus incidence of metabolic diseases including various diseases such as obesity, diabetes, hyperlipidemia, non-alcoholic fatty liver disease, and dyslipidemia is rapidly increasing.

Obesity refers to a condition where excess energy is accumulated in the body fat, as energy intake exceeds energy expenditure. The pharmacological mechanisms of anti-obesity drugs currently used may be largely divided into 1) inhibition of fat absorption, 2) promotion of fat breakdown and heat generation, 3) control of appetite and satiety, 4) inhibition of protein metabolism, and 5) regulation of emotions related to food intake. Representative anti-obesity drugs may include Xenical™ which inhibits fat absorption, and Reductil™ which suppresses appetite by stimulating the sympathetic nervous system. However, Xenical™ has been reported to have side effects such as oily stool, abdominal pain, vomiting, itching, liver damage, etc., and Reductil™ has been reported to have side effects such as headache, loss of appetite, insomnia, constipation, etc., as well as serious cardiovascular side effects.

Diabetes is a disease caused by deficiency or abnormality of insulin secretion, and is characterized by symptoms of elevated blood glucose levels (hyperglycemia) and urinary glucose excretion. Antidiabetic drugs currently used include PPAR-y activators, GLP-1 derivatives, DPP-IV inhibitors, etc., but these traditional drugs have been reported to have side effects such as weight gain and toxicity to liver, kidney, muscle, heart, etc.

Hyperlipidemia is known to be a disease in which fat components present more than necessary in the blood accumulate on the walls of blood vessels to cause inflammation, resulting in cardiovascular diseases such as myocardial infarction, stroke, cerebral infarction, etc. Anti-hyperlipidemic drugs currently used include 'statin'-based drugs having HMG-CoA reductase inhibitory activity, but have been reported to have side effects such as toxicity to liver, muscle, etc., when used for a long period of time.

Dyslipidemia refers to a condition in which total cholesterol, LDL cholesterol, or triglyceride level is increased and HDL cholesterol level is decreased in the blood, and is known to increase the risk of cardiovascular diseases such as heart attack, stroke, etc. Anti-dyslipidemic drugs currently used include BAY13-9952 (implitapide) available from Bayer, which is a drug inhibiting activity of microsomal triglyceride transfer protein (MTP). However, these drugs have a problem in that the intracellular mechanisms of action are not clarified and they may cause side effects.

Non-alcoholic fatty liver disease, which is a fatty liver disease caused by factors other than alcohol among fatty liver disease, refers to fatty acid accumulation in the form of triglycerides in more than 5% of the parenchymal cells of the liver. Non-alcoholic fatty liver disease is pathologically classified into simple steatosis and steatohepatitis with inflammation. It is known that non-alcoholic steatohepatitis may progress to serious liver diseases such as hepatitis, hepatic fibrosis, cirrhosis, etc., when left untreated for a long time. Non-alcoholic fatty liver disease has difficulty in developing a treatment method thereof because the causes of the disease have not yet been clearly identified. In particular, since the target organ, liver is an organ in which various detoxification processes occur, it is not easy to develop an effective drug because there is a risk of hepatocyte damage when a drug with increased pharmacological activity is used.

Current development of effective therapeutic agents for obesity or metabolic diseases is insufficient, and therapeutic agents for respective specific diseases have been also reported to have several side effects. Accordingly, there is an urgent need for the development of a safe therapeutic agent having excellent therapeutic efficacy against obesity or metabolic diseases without side effects.

### [Disclosure]

### [Technical Problem]

A main object of the present invention is to provide a composition for preventing, improving, or treating obesity or metabolic diseases, the composition including a combined extract of peach blossom and lotus leaf as an active ingredient.

Specifically, an object of the present invention is to provide a pharmaceutical composition for preventing or treating obesity or metabolic diseases, the pharmaceutical composition including a combined extract of peach blossom and lotus leaf or a fraction thereof as an active ingredient.

Another object of the present invention is to provide a food composition for preventing or improving obesity or metabolic diseases, the food composition including the combined extract of peach blossom and lotus leaf or the fraction thereof as an active ingredient.

Further, still another object of the present invention is to provide a method of treating or preventing obesity or metabolic diseases using the combined extract of peach blossom and lotus leaf.

Furthermore, still another object of the present invention is to provide use of the combined extract of peach blossom and lotus leaf in preparing therapeutic agents for obesity or metabolic diseases.

### [Technical Solution]

To achieve objects of the present invention, the present invention provides a pharmaceutical composition or a food composition, each including a combined extract or a fraction thereof as an active ingredient. Hereinafter, each composition will be descried in detail.

### Pharmaceutical Composition for Preventing or Treating Obesity or Metabolic Diseases

As an aspect to achieve objects of the present invention, the present invention provides a pharmaceutical composition for preventing or treating obesity or metabolic diseases, the pharmaceutical composition including a combined extract of peach blossom and lotus leaf or a fraction thereof as an active ingredient.

As used herein, the term "peach blossom" refers to a flower of a peach tree or a wild peach belonging to the *Rosaceae* family. Peach blossom is a bisexual flower with a diameter of about 2.5 cm to 3.5 cm, and there are usually varieties of light red, white, deep red, etc. Peach blossom is collected during the flowering period of mid-April or early May, and dried in the shade to be used as a herbal medicine. In the present invention, peach blossom may be a flower of a plant having the scientific name of *Prunus persica* or *Prunus davidiana,* but is not limited thereto.

As used herein, the term "peach blossom extract" refers to an extract obtained by extracting peach blossoms or flower buds with water or an organic solvent.

As used herein, the term "lotus leaf" refers to a leaf of lotus belonging to the *Nelumbonaceae* family. Lotus leaf has a semi-circular shape or a striped fan shape, but a circular shape when unfolded, and has a diameter of 20 cm to 50 cm. The leaf edges are flat or wavy. The upper surface of the leaf is dark green or yellowish green, and the lower surface is slightly glossy, and 21 to 22 rows of protruding leaf veins extend from the center of the petiole in all directions. In the present invention, lotus leaf may be a leaf of a plant having the scientific name of *Nelumbo nucifera,* but is not limited thereto.

As used herein, the term "lotus leaf extract" refers to an extract obtained by extracting lotus leaves with water or an organic solvent.

In the present invention, the "combined extract of peach blossom and lotus leaf" includes both of a mixture of the peach blossom extract and the lotus leaf extract and an extract of a mixture of peach blossom and lotus leaf.

In the present invention, the peach blossom extract and the lotus leaf extract in the mixture of the peach blossom extract and the lotus leaf extract may be mixed at a weight ratio of 10:1 to 1:10, preferably 5:1 to 1:5, and more preferably 3:1 to 1:3.

Further, in the present invention, peach blossom and lotus leaf in the mixture of peach blossom and lotus leaf may be mixed at a weight ratio of 10:1 to 1:10, preferably 5:1 to 1:5, and more preferably 3:1 to 1:3.

As used herein, the term "extract" includes a liquid extract itself and an extract of any formulation which may be prepared using the liquid extract, such as a liquid extract obtained by extraction treatment of a natural substance, a diluted or concentrated liquid of the liquid extract, a dried product obtained by drying the liquid extract, and a crude purification product or a purification product of the liquid extract, or a mixture thereof, etc.

A method of preparing the extract may be, but is not particularly limited to, performed according to a method commonly used in the art. Non-limiting examples of the extraction method may include a hot water extraction method, an ultrasonic extraction method, a filtration method, a reflux extraction method, an immersion extraction method, a high temperature and high pressure steam extraction method, etc., and these methods may be performed alone or in a combination of two or more thereof.

In the present invention, a kind of an extraction solvent used in extracting the natural substance is not particularly limited, and any solvent known in the art may be used. Non-limiting examples of the extraction solvent may include water, alcohol, a mixture thereof, etc. When alcohol is used as a solvent, C₁-C₄ alcohol may be preferably used, and methanol or ethanol may be more preferably used. Most preferably, water, 30% to 80% methanol (v/v), 30% to 80% ethanol (v/v) may be used in extraction. The quantity of the extraction solvent may be preferably twice or twenty times the dry weight of the peach blossom or lotus leaf.

According to one embodiment of the present invention, the combined extract of peach blossom and lotus leaf may be prepared by finely cutting each dry product of peach blossom or lotus leaf, putting each product in an extraction vessel, adding water or C₁ or C₂ lower alcohol or a mixture thereof, preferably water, 50% methanol (v/v), 30% ethanol (v/v), or 70% ethanol (v/v), and performing extraction at a predetermined temperature to prepare single extracts, and then mixing the single extracts with each other at an appropriate ratio.

However, this method is only an example, and the method of preparing the combined extract of peach blossom and lotus leaf is not limited thereto, and the combined extract of peach blossom and lotus leaf of the present invention includes all combined extracts of peach blossom and lotus leaf, which are prepared according to methods known in the art or related art. In the preparation method according to the present invention, when the extraction solvent is water, 50% methanol (v/v), 30% ethanol (v/v), or 70% ethanol (v/v), extraction is preferably performed at 100 °C, 80 °C, 85 °C, or 80 °C, respectively. Subsequently, the liquid extract may be further subjected to filtration, concentration under reduced pressure, drying, and pulverization.

In the present invention, 50% methanol extract of peach blossom and lotus leaf refers to about 50% methanol extract, and may include 45% ∼ 55% methanol extract. 30% ethanol extract of peach blossom and lotus leaf refers to about 30% ethanol extract, and may include 25% ∼ 35% ethanol extract. 70% ethanol extract of peach blossom and lotus leaf refers to about 70% ethanol extract, and may include 65% ∼ 75% ethanol extract.

As used herein, the term "fraction" refers to a product obtained by performing fractionation for separating a particular component or a particular group from a mixture including various components.

In the present invention, a fractionation method of obtaining the fraction is not particularly limited, and may be performed according to a method commonly used in the art. Non-limiting examples of the fractionation method may include a method of obtaining a fraction from an extract by treatment with a predetermined solvent, after obtaining the extract by extracting a natural product.

In the present invention, a kind of a fractionation solvent used in obtaining the fraction is not particularly limited, and any solvent known in the art may be used. Non-limiting examples of the fractionation solvent may include polar solvents such as water, alcohols, etc.; and non-polar solvents such as hexane, ethyl acetate, chloroform, dichloromethane, etc. These solvents may be used alone or in a combination of two or more thereof. Among the fractionation solvents, when alcohol is used, C₁-C₄ alcohol may be preferably used.

The present inventors first identified that the combined extract of peach blossom and lotus leaf has remarkable therapeutic effects on metabolic diseases including obesity, diabetes, hyperlipidemia, dyslipidemia, and non-alcoholic fatty liver disease. In particular, according to embodiments of the present invention, the combined extract of peach blossom and lotus leaf was confirmed to have excellent weight reduction and fat inhibition effects on high-fat-diet fed mice, as compared with the single extract of peach blossom or lotus leaf (FIGS. 1 and 2), and excellent inhibitory effect on blood factors observed in metabolic diseases such as obesity, diabetes, hyperlipidemia, non-alcoholic fatty liver disease, dyslipidemia, etc. (FIG. 3).

As used herein, the term "metabolic diseases" collectively refer to diseases caused by metabolic abnormalities of sugars, lipids, proteins, vitamins, minerals, etc. Specific examples thereof may include obesity and diabetes caused by blood glucose metabolic abnormalities, and hyperlipidemia, dyslipidemia, and non-alcoholic fatty liver disease caused by lipid metabolic abnormalities, etc.

The disease to be prevented or treated in the present invention includes all obesity or metabolic diseases, but may be more preferably selected from the group consisting of obesity and diabetes caused by blood glucose metabolic abnormalities, and hyperlipidemia, dyslipidemia, and non-alcoholic fatty liver disease.

As used herein, the term "obesity" generally refers to a condition of weight gain, that is, a condition of excess fat accumulation in the body, and is pathologically defined as a body mass index (weight (kg) divided by height (m) squared) of 25 or more in Korea and 30 or more according to World Health Organization (WHO). In most cases, obesity refer to a body weight that exceeds the normal weight. However, even if not overweight, when a fat proportion in the body composition is high, it is diagnosed as obesity. Obesity is a disease that occurs in both adults and children. It is known that in individuals with obesity symptoms, fatty acids and glucose, which enter from plasma into fat cells, are usually esterified to accumulate in the form of triglycerides.

As used herein, the term "diabetes" is a disease that is caused by a lack of insulin secretion or abnormal function, and is characterized by symptoms of elevated blood glucose levels (hyperglycemia) and urinary glucose excretion, leading to retinopathy, renal dysfunction, neuropathy, cardiovascular disorders, etc.

As used herein, the term "hyperlipidemia" refers to a disease in which fat components present more than necessary in the blood accumulate on the walls of blood vessels to cause inflammation, resulting in cardiovascular diseases, and it is known that hyperlipidemia itself does not exhibit any special symptoms, but symptoms due to complications may occur. For example, it is known that elevated triglycerides in the blood may lead to pancreatitis, xanthoma in the Achilles tendon, or xanthelasma in the eyelids.

As used herein, the term "dyslipidemia" refers to a condition in which total cholesterol, LDL cholesterol, or triglyceride level is increased or HDL cholesterol level is decreased in the blood, and is known to increase the risk of cardiovascular diseases such as heart attack, stroke, etc. It is known that dyslipidemia may be caused by smoking, drinking, etc., and may also be caused by genetic factors. In the case of diseases such as obesity, diabetes, hypertension, etc., the risk of dyslipidemia is known to increase.

As used herein, the term "non-alcoholic fatty liver disease", which is a fatty liver disease caused by factors other than alcohol among fatty liver disease, refers to fatty acid accumulation in the form of triglycerides in more than 5% of the parenchymal cells of the liver. Non-alcoholic fatty liver disease is pathologically classified into simple steatosis and steatohepatitis with inflammation. It is known that non-alcoholic fatty liver disease may progress to serious liver diseases such as hepatitis, hepatic fibrosis, cirrhosis, etc., when left untreated for a long time.

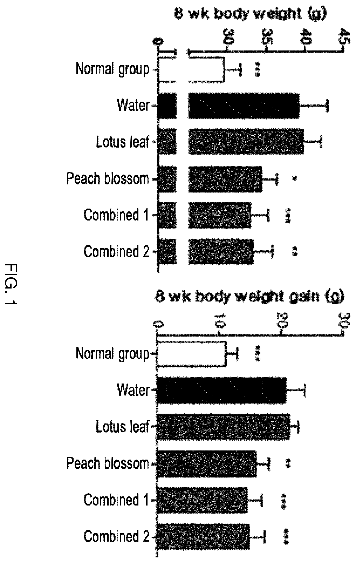

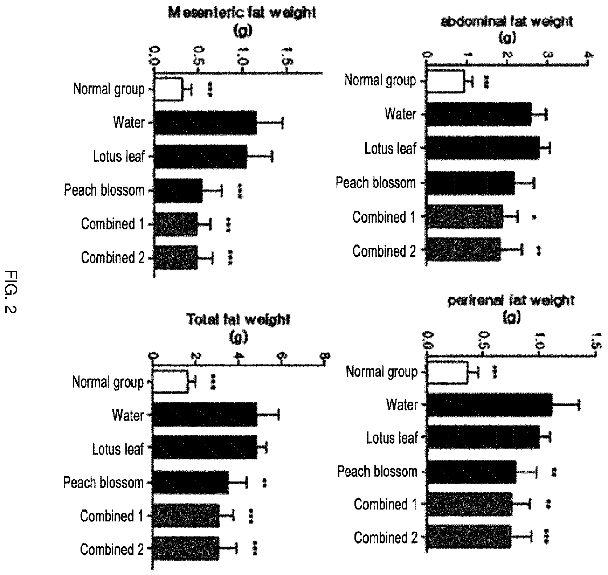

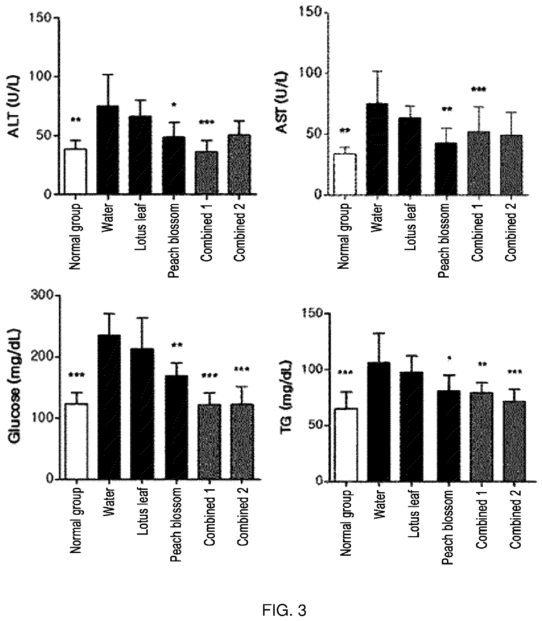

The combined extract of the present invention exhibits remarkably excellent fat inhibitory effect (FIGS. 1 and 2) and also exhibits inhibitory effects on blood factors associated with metabolic diseases such as obesity, diabetes, hyperlipidemia, non-alcoholic fatty liver disease, dyslipidemia, etc. (FIG. 3), as compared with the single extract of peach blossom or lotus leaf. Therefore, the combined extract of peach blossom and lotus leaf of the present invention may be usefully applied to preventing or treating obesity or metabolic diseases.

The pharmaceutical composition including the combined extract of the present invention or the fraction thereof may further include an appropriate carrier, excipient, or diluent commonly used in the preparation of pharmaceutical compositions. In this regard, the amount of the combined extract or the fraction included in the composition is not particularly limited, but the combined extract or the fraction is preferably included in an amount of 10% by weight to 70% by weight with respect to the total weight of the composition, but is not limited thereto.

Further, the composition including the combined extract of the present invention or the fraction thereof may be used after being formulated, according to a common method, into a formulation for oral administration, such as powder, granules, a tablet, a capsule, a suspension, an emulsion, a syrup, an aerosol, etc., a solution for external use, a suppository, and a sterile injectable solution. The carrier, excipient, and diluent which may be included in the composition of the present invention may include lactose, dextrose, sucrose, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, and mineral oil. For formulation, the composition may be formulated using diluents or excipients commonly used, such as fillers, extenders, binders, wetting agents, disintegrating agents, surfactants, etc. Solid formulations for oral administration may include tablets, pills, powders, granules, capsules, etc., and these solid formulations are prescribed by mixing the combined extract or fraction thereof with at least one excipient, for example, starch, calcium carbonate, sucrose, lactose, gelatin, etc. Moreover, lubricants such as magnesium stearate, talc, etc. may be used in addition to simple excipients. Liquid formulations for oral administration may include suspensions, liquids for internal use, emulsions, syrups, etc., and various excipients, for example, wetting agents, sweeteners, flavoring agents, preservatives, etc. may be used in addition to simple diluents commonly used, such as water, liquid paraffin, etc. Formulations for parenteral administration may include sterilized aqueous solutions, non-aqueous solvents, suspensions, emulsions, freeze-dried preparations, and suppositories. Examples of the non-aqueous solvents and suspensions may include propylene glycol, polyethylene glycol, vegetable oils such as olive oil, injectable esters such as ethyl oleate, etc. Examples of suppository bases may include Witepsol, macrogol, Tween 61, cocoa butter, laurin butter, glycerogelatin, etc.

The preferred administration dose of the combined extract of the present invention may vary depending on a patient's conditions and body weight, severity of the disease, the form of drug, and administration route and duration, and may be appropriately selected by those skilled in the art. Administration may be performed once a day or in equally divided doses.

The pharmaceutical composition of the present invention may be administered via various routes to mammals such as rats, mice, livestock, humans, etc. Any mode of administration may be contemplated, for example, oral, rectal, intravenous, intramuscular, subcutaneous injection, etc.

### Food Composition for Preventing or Improving Obesity or Metabolic Diseases

As another aspect to achieve objects of the present invention, the present invention provides a food composition for preventing or improving obesity or metabolic diseases, the food composition including a combined extract of peach blossom and lotus leaf or a fraction thereof as an active ingredient.

Since the combined extract of peach blossom and lotus leaf of the present invention or the fraction thereof was derived from natural products, and safety thereof was demonstrated, it may also be used as a food composition.

The food composition including the combined extract of peach blossom and lotus leaf of the present invention or the fraction thereof as an active ingredient has excellent weight loss efficacy and exhibits excellent efficacy of improving obesity or metabolic diseases.

The combined extract of peach blossom and lotus leaf of the present invention or the fraction thereof is preferably included in an amount of 10% by weight to 70% by weight with respect to the total weight of the composition, but is not limited thereto.

The food composition of the present invention may include an additional ingredient that is commonly used in food compositions so as to improve smell, taste, vision, etc. For example, the composition may include vitamins A, C, D, E, B1, B2, B6, B12, niacin, biotin, folate, pantothenic acid, etc. Additionally, the composition may also include minerals such as Zinc (Zn), iron (Fe), calcium (Ca), chromium (Cr), magnesium (Mg), manganese (Mn), copper (Cu), etc. Additionally, the composition may also include amino acids such as lysine, tryptophan, cysteine, valine, etc. Additionally, the composition may also include food additives, such as preservatives (potassium sorbate, sodium benzoate, salicylic acid, sodium dehydroacetate, etc.), disinfectants (bleaching powder, higher bleaching powder, sodium hypochlorite, etc.), antioxidants (butylhydroxyanisole (BHA), butylhydroxytoluene (BHT), etc.), coloring agents (tar color, etc.), color-developing agents (sodium nitrite, etc.), bleaching agents (sodium sulfite), seasonings (monosodium glutamate (MSG), etc.), sweeteners (dulcin, cyclemate, saccharin, sodium, etc.), flavors (vanillin, lactones, etc.), swelling agents (alum, potassium D-hydrogen tartate, etc.), fortifiers, emulsifiers, thickeners (adhesive pastes), film-forming agents, gum base agents, antifoaming agents, solvents, improvers, etc. The additives may be selected and used in an appropriate amount according to the food types.

In one embodiment of the present invention, the food composition may be a health functional food or a health supplement food.

As used herein, the term "health food" refers to a food having an effect of actively maintaining or promoting health, compared to a general food, and the "health supplement food" refers to a food for health supplement. In some cases, the terms "functional food", "health food", and "health supplement food" are used interchangeably. The food may be prepared in various forms, such as tablets, capsules, powders, granules, liquids, pills, etc., to obtain useful effects.

As used herein, the term "functional food" is the same term as food for special health use (FoSHU), and refers to a food having high medicinal and medical effects, which is processed to effectively exert a body-regulating function as well as to supply nutrients.

As specific examples of the health functional food of the present invention, the combined extract of peach blossom and lotus leaf or the fraction thereof may be used to provide processed foods that are modified to have improved storage while maintaining properties of agricultural products, livestock products, or marine products. The health functional food including the combined extract of peach blossom and lotus leaf or the fraction thereof may be, but is not particularly limited to, preferably those prepared in the form of margarines, fat continuous or water continuous or bicontinuous spreads, fat reduced spreads, confectionery products such as chocolate or chocolate coatings or chocolate fillings or bakery fillings, ice creams, ice cream coatings, ice cream inclusions, dressings, mayonnaise, cheeses, cream alternatives, dry soups, drinks, cereal bars, sauces, snack bars, dairy products, clinical nutrition products, infant formulations, etc.

### Use of Combined Extract of Peach Blossom and Lotus Leaf or Fraction Thereof, and Treatment Method of Using Combined Extract of Peach Blossom and Lotus Leaf or Fraction Thereof

The present invention provides use of the combined extract of peach blossom and lotus leaf or the fraction thereof in preparing a therapeutic agent for obesity or metabolic diseases.

Further, the present invention provides a method of preventing, improving, or treating obesity or metabolic diseases, the method including the step of administering a therapeutically effective amount of the combined extract of peach blossom and lotus leaf or the fraction thereof to a subject in need of preventing or treating obesity or metabolic diseases. In the present invention, the subject may be a mammal, particularly, a human.

### [Advantageous Effects]

A combined extract of peach blossom and lotus leaf of the present invention or a fraction thereof has excellent efficacy against obesity or metabolic diseases and has no side effects because it is derived from natural products, and therefore, it may be variously applied to preventing, improving, or treating obesity or metabolic diseases.

### [Description of Drawings]

FIG. 1 is a graph showing a comparison of total weight and weight gain in high-fat-diet fed mouse models, each mouse model fed with a single extract of peach blossom (indicated by Peach blossom), a single extract of lotus leaf (indicated by Lotus leaf), or a combined extract of the present invention (indicated by Combined 1 or Combined 2);
FIG. 2 is a graph showing a comparison of abnominal fat weight, perirenal fat weight, mesentric fat weight, and total fat weight in high-fat-diet fed mouse models, each mouse model fed with a single extract of peach blossom (indicated by Peach blossom), a single extract of lotus leaf (indicated by Lotus leaf), or a combined extract of the present invention (indicated by Combined 1 or Combined 2); and
FIG. 3 is a graph showing a comparison of liver enzyme levels (ALT and AST), glucose levels, and triglyceride (TG) levels in high-fat-diet fed mouse models, each mouse model fed with a single extract of peach blossom (indicated by Peach blossom), a single extract of lotus leaf (indicated by Lotus leaf), or a combined extract of the present invention (indicated by Combined 1 or Combined 2).

### [Mode for Invention]

Hereinafter, the present invention will be described in more detail with reference to Preparation Examples and Examples. However, these Preparation Examples and Examples are for illustrative purposes only, and the scope of the present invention is not intended to be limited by these Preparation Examples and Examples.

### <Preparation Example> Preparation of Single Extract or Combined Extract

### Preparation Example 1: Preparation of single extract of peach blossom

A dry product of peach blossom was finely cut, and then put in an extraction vessel. Water was added thereto, followed by reflux extraction twice at 100 °C for 3 hr. This liquid extract was filtered using a filter paper, and then concentrated under reduced pressure using a rotary vacuum evaporator (EYELA, japan), and then freeze-dried using a freeze dryer (OPR-FDY-8612, OPERON, KOREA) to prepare a single extract of peach blossom.

### Preparation Example 2: Preparation of single extract of lotus leaf

A dry product of lotus leaf was finely cut, and then extracted and freeze-dried in the same manner as in Preparation Example 1 to prepare a single extract of lotus leaf.

### Preparation Example 3: Preparation of combined extract of peach blossom and lotus leaf of the present invention

The single extract of peach blossom prepared in Preparation Example 1 and the single extract of lotus leaf prepared in Preparation Example 2 were mixed at a weight ratio as in the following Table to prepare a combined extract of the present invention.

**[Table 1]**

| Preparation Example | Peach blossom | Lotus leaf |
|---|---|---|
| 3-1 | 1 | 1 |
| 3-2 | 2 | 1 |

### Preparation Example 4: Preparation of combined extract of peach blossom and lotus leaf of the present invention

Each dry product of peach blossom and lotus leaf was finely cut, and then mixed at a weight ratio as in the following Table 2, and extracted and freeze-dried in the same manner as in Preparation Example 1 to prepare a combined extract of the present invention.

**[Table 2]**

| Preparation Example | Peach blossom | Lotus leaf |
|---|---|---|
| 4-1 | 1 | 1.35 |
| 4-2 | 1 | 0.65 |

### Example 1: Examination of body fat reduction effect in high-fat-diet fed mouse model

To examine the weight loss effect of the combined extract of peach blossom and lotus leaf of the present invention, 21-day-old (3-week-old) male mice C57BL/6 were purchased and acclimated with free to access water and feed (chow diet) for 1 week. When the experimental animals were 4 weeks of age, the weights of the experimental animals were measured and arranged so that the average weight deviation per cage was evenly within 0.1 g, and divided into a total of 6 groups (n = 8 for each group) by varying diet conditions as in the following Table 3. Thereafter, a high-fat feed (60% kcal fat) mixed with the extract corresponding to each group of the following Table 3 was fed every 3 to 4 days for a total of 8 weeks.

**[Table 3]**

| | Feed | Feeding of extract | Feeding method | Animal (n) |
|---|---|---|---|---|
| Normal group | Standard feed | - | Free access | 8 mice |
| Control group (water) | 60% fat contained | - | Free access | 8 mice |
| Preparation Example 1 (Peach blossom) | 60% fat contained | 2 g/kg diet | Free access | 8 mice |
| Preparation Example 2 (Lotus leaf) | 60% fat contained | 2 g/kg diet | Free access | 8 mice |
| Preparation Example 3-1 (combined 1) | 60% fat contained | 2 g/kg diet | Free access | 8 mice |
| Preparation Example 3-2 (combined 2) | 60% fat contained | 2 g/kg diet | Free access | 8 mice |

As a result, the mouse models fed with the combined extract of peach blossom and lotus leaf of the present invention exhibited excellent weight loss effect, as compared with the mouse models fed with the single extract of peach blossom. In particular, even though the single extract of lotus leaf exhibited no weight loss effect, the combined extract of peach blossom and lotus leaf, in which the amount of peach blossom was reduced to 1/2 of the single extract of peach blossom, exhibited better weight loss effect than the single extract of peach blossom (FIG. 1).

Meanwhile, to examine that the weight loss effect was fat inhibitory effect, fat mass of each body area of the experimental animals was measured and recorded. In detail, after completion of the experiment, abdominal fat, perirenal fat, and mesentric fat of the experimental animals were removed and weighed.

As a result, it was confirmed that the mouse models fed with the combined extract of peach blossom and lotus leaf of the present invention exhibited excellent inhibitory effects on fat mass of all body areas, as compared with the mouse models fed with the single extract of peach blossom or the single extract of lotus leaf (FIG. 2).

These results suggest that the combined extract of peach blossom and lotus leaf of the present invention exhibits remarkably excellent fat inhibitory effects, as compared with the single extract of peach blossom or the single extract of lotus leaf, and thus it may be usefully applied to preventing or treating metabolic diseases such as obesity, etc.

### Example 2: Examination of reduction effect of obesity or metabolic disease markers in blood of high-fat-diet fed mouse model

To further examine that the combined extract of peach blossom and lotus leaf of the present invention is effective in preventing or treating metabolic diseases, liver alanine aminotransferase (ALT) and aspartate aminotransferase (AST) levels, blood glucose levels, and triglyceride (TG) levels were measured in the plasma separated from the blood which was collected before removing the fat tissues of the mice used in Example 1.

As a result, when high-fat-diet fed mouse models were fed with the combined extract of peach blossom and lotus leaf of the present invention, the combined extract showed remarkably increased inhibitory effects on all the blood ALT and AST levels, blood glucose levels, and triglyceride (TG) levels, which are chronically elevated in metabolic diseases such as obesity, diabetes, hyperlipidemia, dyslipidemia, non-alcoholic fatty liver disease, etc., as compared with the mouse models fed with the single extract of peach blossom or the single extract of lotus leaf (FIG. 3).

These results suggest that the combined extract of peach blossom and lotus leaf of the present invention exhibits remarkably increased therapeutic effects on metabolic diseases, as compared with the single extract of peach blossom or the single extract of lotus leaf, and thus it may be usefully applied to preventing or treating metabolic diseases such as obesity, diabetes, hyperlipidemia, dyslipidemia, non-alcoholic fatty liver disease, etc.

Since contents may be sufficiently recognized and inferred by those skilled in the art, a detailed description thereof will be omitted. In addition to the specific examples described in the present specification, various modifications are possible without changing the technical spirit or essential configuration of the present invention. Therefore, the present invention may be implemented in a different manner from those specifically described and exemplified in this specification, which is understood by any person skilled in the technical field of the present invention.

## Claims

1. A pharmaceutical composition for preventing or treating obesity or metabolic diseases, the pharmaceutical composition comprising a combined extract of peach blossom and lotus leaf or a fraction thereof as an active ingredient.

2. The pharmaceutical composition of claim 1, wherein the combined extract is a mixture of a peach blossom extract and a lotus leaf extract, or an extract obtained by extracting a mixture of peach blossom and lotus leaf.

3. The pharmaceutical composition of claim 2, wherein the combined extract is extracted using water, C₁-C₄ alcohol, or a mixture thereof.

4. The pharmaceutical composition of claim 1, wherein the pharmaceutical composition is used for preventing or treating obesity.

5. The pharmaceutical composition of claim 1, wherein the pharmaceutical composition is used for preventing or treating metabolic diseases, and the metabolic diseases are selected from the group consisting of diabetes, hyperlipidemia, dyslipidemia, and non-alcoholic fatty liver disease.

6. The pharmaceutical composition of claim 1, wherein the combined extract or the fraction thereof has a weight loss effect.

7. A food composition for preventing or improving obesity or metabolic diseases, the food composition comprising a combined extract of peach blossom and lotus leaf or a fraction thereof as an active ingredient.

8. The food composition of claim 7, wherein the composition is a health functional food or a health supplement food.

9. A method of preventing or treating obesity or metabolic diseases, the method comprising the step of administering a therapeutically effective amount of a combined extract of peach blossom and lotus leaf or a fraction thereof to a subject in need of preventing or treating obesity or metabolic diseases.

10. Use of a combined extract of peach blossom and lotus leaf or a fraction thereof in preparing a prophylactic or therapeutic agent for obesity or metabolic diseases.
